# EUROPÄISCHE PATENTSCHRIFT

(11) **EP 0 434 936 B1**
(45) Veröffentlichungstag und Bekanntmachung des Hinweises auf die Patenterteilung: **29.01.1997**
(21) Anmeldenummer: 90121049.2
(22) Anmeldetag: 02.11.1990
(51) Int. Cl.: A61B 8/00, A61B 17/22

(54) **Halterung eines diagnostischen Ultraschall-Transducers**
Support provided for an ultrasonic diagnostic transducer
Support pour transducteur diagnostique à ultrasons

(30) Priorität: 21.12.1989 DE 3942253
(43) Veröffentlichungstag der Anmeldung: 03.07.1991
(73) Patentinhaber: DORNIER MEDIZINTECHNIK GMBH, D-82101 Germering (DE)
(72) Erfinder: Denk, Roland, Dipl.-Phys., W-8000 München 40 (DE); Hesse, Alexander, Dr.-Ing., W-8000 München 19 (DE); Haas, Anton, Dipl.-Ing., W-8000 München 82 (DE); Kreibich, Rainer, W-8000 München 19 (DE); Viebach, Thomas, Dipl.-Ing., W-8081 Pischertshofen (DE)
(74) Vertreter: Landsmann, Ralf, Dipl.-Ing.

(56) Entgegenhaltungen:
- EP-A- 0 083 973
- DE-A- 3 608 877
- DE-U- 1 860 245
- DE-U- 8 809 253

## Beschreibung

Die Erfindung betrifft einen innerhalb der Apertur einer wassergefüllten Stosswellenquelle befindlichen Ultraschall-Transducer für die Diagnose von Konkrementen im Körper von Lebewesen.

Transducer dieser Art sind beispielsweise aus dem Buch "End of the Stone Age" London 1987 bekannt. Transducer werden sowohl bei Stosswellenquellen mittels Funkenstrecke wie auch bei elektromagnetisch erzeugten Stosswellen (EMSE) und bei piezo-keramischen Stosswellenquellen verwendet.

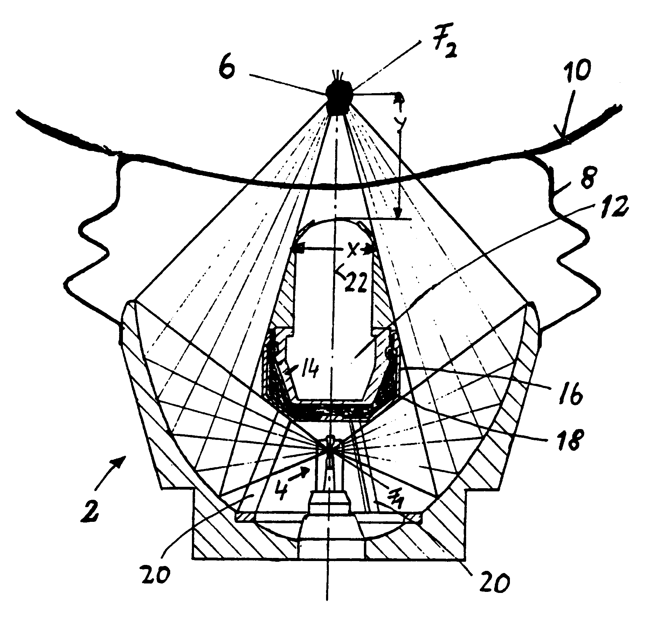

In der Figur 5 des Dokumentes DE-A-3 608 877 wird eine Stoßwellenquelle mit einer Halterung für einen Ultraschallwandler gezeigt. Zum Schutze des Ultraschallwandlers sind auf dessen Halterung Dämpfungsschichten angebracht , wobei die äußere Schicht direkt an das Stoßwellenausbreitungsmedium angrenzt und wobei die innere Schicht direkt auf dem Wandler aufgebracht ist.

Aufgabe der Erfindung ist es, die Beanspruchung eines Ultraschall-Transducers in der Apertur einer Stosswellenquelle zu vermindern und damit die Standzeit des Transducers zu erhöhen.

Diese Aufgabe wird erfindungsgemäss dadurch gelöst, dass die Halterung des Ultraschall-Transducers in einer wassergefüllten Apertur aus einer metallischen Aussenschale, einer metallischen Innenschale jeweils mit grosser Impedanz-differenz zu Wasser und dazwischen befindlicher Dämpfungsschicht mit stark unterschiedlicher akustischer Impedanz zu den metallischen Schalen besteht.

Bei einer vorteilhaften Ausgestaltung der Erfindung ist die Dicke der Dämpfungsschicht so gewählt, dass der Frequenzanteil des Stosswellenspektrums mit der maximalen Intensität durch Interferenz ausgelöscht wird.

Die Erfindung wird nachfolgend anhand einer Figur, die eine Querschnittsansicht eines Ausführungsbeispiels zeigt, beschrieben.

Die Figur zeigt eine an sich bekannte Stosswellenquelle mit einem Reflektor 2, der Teil eines Rotationsellipsoids ist und eine Funkenstrecke 4 im Brennpunkt F1. Im zweiten Brennpunkt F2 befindet sich ein zu zerstörendes Konkrement 6. Das wassergefüllte Rotationsellipsoid ist mit einem Wassersack 8 verschlossen, der luftspaltlos an der Haut 10 eines Patienten anliegt.

Um die Beanspruchung eines Ultraschall-Transducers 12 in der Apertur des Ellipsoids durch die Stosswelle zu reduzieren, wurde die erfindungsgemässe Halterung entwickelt.

Die Aufnahme des Transducers 12 ist zweischalig mit einem dazwischenliegenden Dämpfungsmedium ausgeführt.
Die Innenschale 14 und die Aussenschale 16 sind aus Metall mit grosser Impedanzdifferenz zu Wasser. Das Dämpfungsmedium 18 besteht aus mit gashaltigen Füllstoffen versehenen oder geschäumten Kunststoff, Gummi oder Silikonkautschuk.

Dabei ergeben sich für die Stosswelle auf dem Weg zum Transducer 12 vier Grenzflächen an Medien mit stark unterschiedlicher akustischer Impedanz. Die Innenschale 14 ist von der Aussenschale 16 und der Befestigung 20 mechanisch weitgehend entkoppelt.

Die Stosswelle durchläuft folgende Grenzflächen:
Wasser - Metall
Metall - Dämpfungsmedium
Dämpfungsmedium - Metall
Metall - Ultraschall-Transducer.

An diesen vier Grenzflächen mit unterschiedlicher akustischer Impedanz erfährt die Stosswelle jeweils eine Teilreflexion, was insgesamt zu einer starken Abschwächung des auf den Transducer 12 einwirkenden Stosswellenanteils führt. Die Dicke der Dämpfungsschicht 18 wurde so gewählt, dass der Frequenzanteil des Stosswellenspektrums mit der maximalen Intensität durch Interferenz ausgelöscht wird.

Die hohe innere Dämpfung des verwendeten Dämpfungsmediums 18, einem Gemisch aus Zwei-Komponentensilikon und microspheres (Hohlglaskugeln), resultiert aus der Vielzahl der statisch verteilten Kunststoff-Gas-Grenzflächen, an denen die Stosswellenfront diffus reflektiert wird.

Da der Ultraschall-Transducer 12 zur Ortung des Steins dient, muß eine exakte Ausrichtung seiner Mittelachse 22 auch bei Krafteinwirkung auf das Gehäuse gewährleistet sein.
Das als Dämpfungsmedium verwendete Kunststoffmaterial muß daher so ausgeführt sein, dass durch seine mechanische Steifigkeit eine formstabile Einbettung des In-Line-Transducers gegeben ist und auch beim direkten Ankoppeln an den Patientenkörper keine störenden Verschiebungen bzw. Verformungen des Ortungssystems auftreten.

Konstruktiv wurde dafür Sorge getragen, dass die Abschattung der reflektierten Stosswelle durch die In-Line-Transducererhaltung minimiert wird. Die Aussenkontur wird durch den Durchmesser (x) des Ultraschall-Transducers und den Abstand (y) von dem Therapiefokus F2 bestimmt. Dadurch wird die Stosswelle entlang des Schutzmantels geführt ("wave guide"). Die Stosswellenfront läuft senkrecht am Schutzmantel entlang; das heisst es gibt keine Reflexionen und minimale Beugung, die Stosswelle wird nur geringfügig geschwächt.

Durch die Abschattung der Stosswellenanteile, die in der Apertur nahe der Rotationsachse 22 (z-Achse) des Ellipsoids liegen, werden phasenstörende Anteile (keine optimale Reflektion im Elektrodenbereich) ausgeblendet und die Fokussierung wird gegenüber einem Ellipsoids ohne In-Line Halterung verbessert.

Durch die In-Line Halterung wird die Primärwelle vollständig abgeschattet.

## Patentansprüche

1. Innerhalb der Apertur einer wassergefüllten Stoßwellenquelle (2,4) befindlicher Ultraschall-Transducer (12), der in einer Halterung befestigt ist, welche, von der Stoßwellenquelle aus gesehen, eine Schichtenfolge aus einer Dämpfungsschicht und einer stoßwellenreflektierenden Schicht (14) aufweist, **dadurch gekennzeichnet,** daß die stoßwellenreflektierende Schicht als eine direkt am Ultraschall-Wandler anliegende metallische Innenschale (14) ausgebildet ist, und daß die Dämpfungsschicht (18) zur Stoßwellenquelle hin von einer metallischen Außenschale (16) umschlossen ist und im Vergleich zu den metallischen Schalen (14, 16), welche eine große akustische Impedanzdiffererz zu Wasser aufweisen, eine stark unterschiedliche akustische Impedanz besitzt.

## Claims

1. An ultrasonic transducer (12), which is situated inside the aperture of a shock wave source (2, 4) filled with water, and which is fixed in a mounting which, as seen from the shock wave source, has a layer sequence comprising a damping layer and a shock wave-reflecting layer (14), **characterised in that** the shock wave-reflecting layer is constructed as a metallic inner shell (14) seated directly against the ultrasonic transducer, and that the damping layer (18) is surrounded by a metallic outer shell (16) towards the shock wave source and has an acoustic impedance which is pronouncedly different from that of the metallic shells (14, 16), which exhibit a large difference in acoustic impedance in relation to that of water.

## Revendications

1. Transducteur (12) à ultrasons disposé dans l'ouverture d'une source (2, 4) d'ondes de choc remplie d'eau et fixé à un support qui, vu depuis la source d'ondes de choc, présente un succession de couches avec une couche amortissante et une couche (14) réfléchissant les ondes de choc, caractérisé par le fait que la couche réfléchissant les ondes de choc se présente sous la forme d'une coque (14) métallique intérieure appliquée directement sur le transducteur à ultrasons et par le fait que la couche amortissante (18), coté source d'ondes de choc, est entourée d'une coque (16) métallique extérieure et possède une impédance acoustique nettement différenciée vis-à-vis des coques métalliques (14, 16) qui présentent une grande différence d'impédance acoustique par rapport à l'eau.
